(19) Europäisches Patentamt
European Patent Office
Office européen des brevets

(11) Publication number : **0 643 053 A1**

(12) **EUROPEAN PATENT APPLICATION**

(21) Application number : **94810470.8**

(22) Date of filing : **16.08.94**

(51) Int. Cl.[6] : **C07D 317/22,** A01N 43/28, C07C 43/295, A01N 31/14

(30) Priority : **23.08.93 CH 2503/93**

(43) Date of publication of application : **15.03.95 Bulletin 95/11**

(84) Designated Contracting States : **AT BE CH DE DK ES FR GB GR IE IT LI NL PT SE**

(71) Applicant : **CIBA-GEIGY AG**
**Klybeckstrasse 141**
**CH-4002 Basel (CH)**

(72) Inventor : **Barz, Dr. Michael**
**Föhrenstrasse 5**
**CH-4313 Möhlin (CH)**
Inventor : **Karrer, Dr. Friedrich**
**Rebbergstrasse 5**
**CH-4800 Zofingen (CH)**

(54) **Process for the manufacture of phenoxyphenoxyalkyl derivatives.**

(57) The invention relates to a process for the manufacture of a compound of the formula

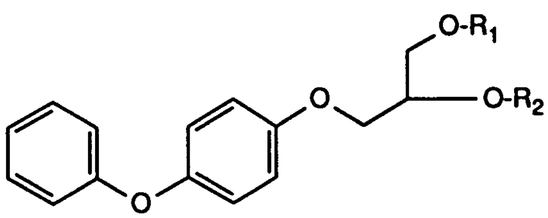

(I),

in which either $R_1$ is hydrogen and $R_2$ is hydrogen or O-$R_1$ and O-$R_2$ taken together are the group of the formula

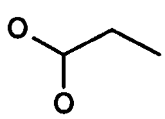

(II),

a compound of the formula I containing the group of the formula II, that is to say a 2-ethyl-4-(4-phenoxyphenoxymethyl)-1,3-dioxolane of the formula

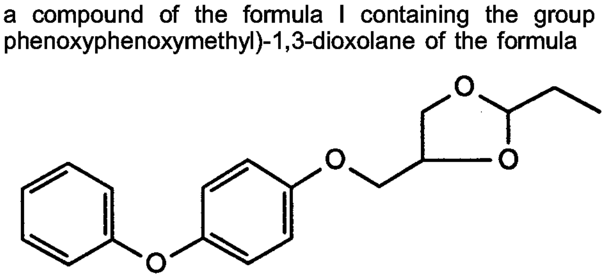

(III),

being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId,
which process is characterised in that
a) for the manufacture of a compound of the formula I, in which $R_1$ is hydrogen and $R_2$ is hydrogen, that is to say of a compound of the formula

EP 0 643 053 A1

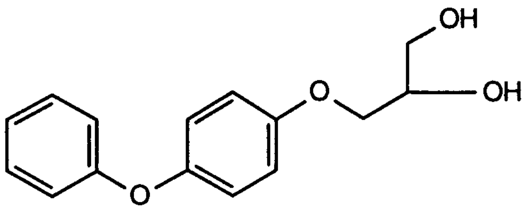

(IV),

the compound of the formula

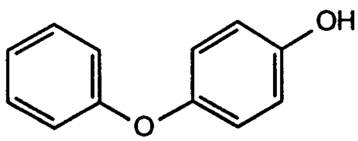

(V)

is reacted with a compound of the formula

$$Y-CH_2-CH(OH)-CH_2-OH \quad (VI),$$

in which Y is a leaving group, or

b) for the manufacture of a compound of the formula III a compound of the formula IV is reacted with the compound of the formula

$$H_3C-CH_2-C(=O)-H \quad (VII)$$

in the presence of an acid catalyst and without complete removal of the water formed during the reaction, the molar ratio, in which the two reactants are used, being defined as the quotient "number of moles used of the compound of the formula IV : number of moles used of the compound of the formula VII", being 1 or less than 1, or

c) for the manufacture of a compound of the formula III the compound of the formula V is reacted with a compound of the formula VI, in which Y is a leaving group, to give a compound of the formula IV, and this compound of the formula IV, without being isolated, is reacted with the compound of the formula VII in the presence of an acid catalyst and without complete removal of the water formed during the reaction, the molar ratio, in which the two reactants are used, being 1 or less than 1,

and to a compound of the formula III and its use as active ingredient in the field of pest control.

The invention relates to a process for the manufacture of a compound of the formula

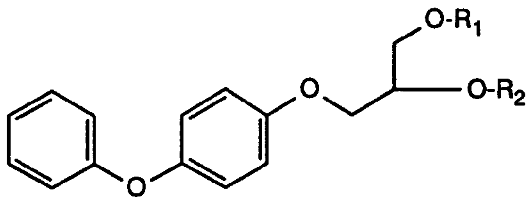

(I),

in which
either $R_1$ is hydrogen and $R_2$ is hydrogen
or $O-R_1$ and $O-R_2$ taken together are the group of the formula

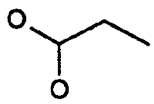

(II),

a compound of the formula I containing the group of the formula II, that is to say a 2-ethyl-4-(4-phenoxyphenoxymethyl)-1,3-dioxolane of the formula

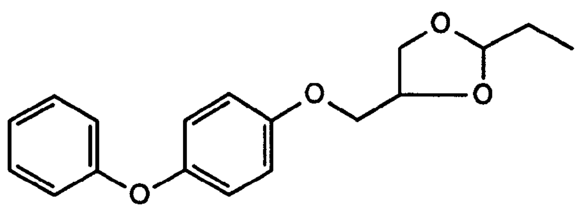

(III),

being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId,
which process is characterised in that

a) for the manufacture of a compound of the formula I, in which $R_1$ is hydrogen and $R_2$ is hydrogen, that is to say of a compound of the formula

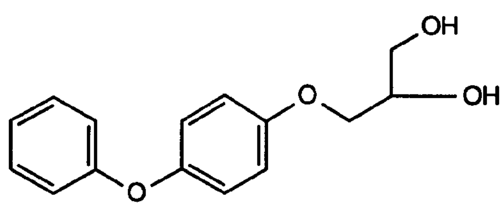

(IV),

the compound of the formula

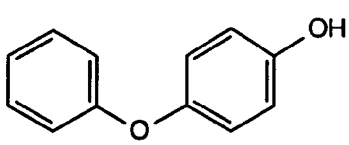

(V)

is reacted, preferably in the presence of a base, with a compound of the formula

$$Y-CH_2-CH(OH)-CH_2-OH \quad (VI),$$

in which Y is a leaving group, or

b) for the manufacture of a compound of the formula III a compound of the formula IV is reacted with the

compound of the formula

$$H_3C\text{-}CH_2\text{-}C(=O)\text{-}H \qquad (VII)$$

in the presence of an acid catalyst and without complete removal of the water formed during the reaction, the molar ratio, in which the two reactants are used, being defined as the quotient "number of moles used of the compound of the formula IV : number of moles used of the compound of the formula VII", being 1 or less than 1, or

c) for the manufacture of a compound of the formula III the compound of the formula V is reacted, preferably in the presence of a base, with a compound of the formula VI, in which Y is a leaving group, to give a compound of the formula IV, and this compound of the formula IV, without being isolated, is reacted with the compound of the formula VII in the presence of an acid catalyst and without complete removal of the water formed during the reaction, the molar ratio, in which the two reactants are used, being defined as the quotient "number of moles used of the compound of the formula IV: number of moles used of the compound of the formula VII", being 1 or less than 1,

to a compound of the formula III, being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId,

to the use of this compound of the formula III,

to a pesticidal composition comprising this compound of the formula III,

to a process for the preparation of this composition,

to the use of this composition,

to plant propagation material treated with this composition and

to a method of controlling pests using this composition.

The reactions described hereinabove and hereinafter are carried out, as required, with cooling, at room temperature or with heating and/or under normal or elevated pressure or under vacuum, optionally in a sealed container and/or in an inert gas atmosphere.

Particularly advantageous reaction conditions are described hereinafter and can especially be found in the Examples.

Variant a)

Suitable leaving groups Y in the compounds VI are, for example, $C_1$-$C_8$alkoxy, such as ethoxy, halo-$C_1$-$C_8$alkoxy, $C_1$-$C_8$alkanoyloxy, such as acetoxy, $C_1$-$C_8$alkylthio, such as methylthio, halo-$C_1$-$C_8$alkylthio, $C_1$-$C_8$alkanesulfonyloxy, such as methane sulfonyloxy, halo-$C_1$-$C_8$alkanesulfonyloxy, such as trifluoromethane sulfonyloxy, benzenesulfonyloxy, toluenesulfonyloxy and halogen, such as fluorine, chlorine, bromine or iodine. Preferred are methane sulfonyloxy, toluenesulfonyloxy and halogen. Especially preferred are chlorine and bromine. Very especially preferred is chlorine.

Suitable bases for facilitating the reaction, that is to say the detachment of HY, are, for example, the oxides, hydroxides, hydrides, amides, alkanolates, acetates, carbonates, bicarbonates, dialkylamides and alkylsilylamides of alkali metals or alkaline earth metals, alkylamines, alkylenediamines, free or N-alkylated, saturated or unsaturated cycloalkylamines, basic heterocycles and ammonium hydroxides. Examples which may be mentioned are calcium oxide, sodium hydroxide, potassium hydroxide, calcium hydroxide, sodium hydride, potassium hydride, calcium hydride, sodium amide, sodium methanolate, potassium tert-butanolate, sodium acetate, sodium carbonate, potassium carbonate, sodium bicarbonate, lithium diisopropylamide, potassium bis(trimethylsilyl)amide, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, 1,5-diazabicyclo[5.4.0]undec-5-ene (DBU) and benzyltrimethylammoniumhydroxide. Preferred are hydroxides, alkanolates, carbonates and bicarbonates of alkali metals or alkaline earth metals, alkylamines and basic heterocycles, such as sodium hydroxide, potassium hydroxide, calcium hydroxide, sodium methanolate, potassium tert-butanolate, sodium carbonate, potassium carbonate, sodium bicarbonate, triethylamine, diisopropylethylamine, pyridine, 4-(N,N-dimethylamino)pyridine, quinuclidine, N-methylmorpholine and 1,5-diazabicyclo[5.4.0]undec-5-ene (DBU). Preferred are also hydroxides, carbonates and bicarbonates of alkali metals or alkaline earth metals. Especially preferred are alkanolates, carbonates and bicarbonates of alkali metals, alkylamines and basic heterocycles, such as sodium methanolate, potassium carbonate, sodium bicarbonate, triethylamine and pyridine. Very especially preferred is potassium carbonate. Bases which are at room temperature in the solid state, are preferably added to the reaction mixture as such, preferably in finely ground form; potassium carbonate, for example, is preferably used in the form of potassium carbonate powder. The

quantity of base, which is used in the reaction, is not critical; however, preferably at least one equivalent, more preferably from 1 to 2 equivalents, most preferably from 1.4 to 1.7 equivalents, of the base is or are used.

The reactants can be reacted with each other as such, i. e. without adding a solvent or diluent, for example in the molten state. However, in most cases it is advantageous to add an inert solvent or diluent or a mixture of these. The following may be mentioned as examples of such solvents or diluents: aromatic, aliphatic and alicyclic hydrocarbons and halohydrocarbons, such as benzene, toluene, xylene, mesitylene, tetralin, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol dimethyl ether, dimethoxydiethyl ether, tetrahydrofuran or dioxane; and water. If the reaction is carried out in the presence of a base, then bases which are employed in excess, such as triethylamine, pyridine, N-methyl-morpholine or N,N-diethylaniline, can also act as solvents or diluents. Preferred are aromatic hydrocarbons and halohydrocarbons. Especially preferred are benzene, toluene, xylene and chlorobenzene. Very especially preferred is toluene. The quantity of the inert solvent or diluent or of the inert solvent or diluent mixture, which is used in the reaction, is not critical; however, preferably a large excess thereof is used. Preferably a catalytic amount of water is used together with one or more of the organic solvents or diluents mentioned. Preferred is the use of benzene, xylene, chlorobenzene and especially toluene, in each case containing a catalytic amount of water.

The molar ratio, in which the two reactants V and VI are used in the reaction, being defined as the quotient "number of moles used of the compound V : number of moles used of the compound VI", is not critical; however, preferably this molar ratio is 1 or less than 1, more preferably from 0.5 to 1, most preferably from 0.8 to 0.95.

The reaction is advantageously carried out in a temperature range from approximately 0°C to the reflux temperature of the reaction mixture, preferably from approximately +50°C to approximately +130°C, in many cases from approximately +70°C to the reflux temperature of the reaction mixture.

The reaction is preferably carried out under normal pressure.

The reaction time is not critical; however, preferably a reaction time in the range from approximately 30 minutes to approximately 10 hours, more preferably from approximately 1 hour to approximately 7 hours, most preferably from approximately 2 hours to approximately 5 hours, is used.

The isolation of the compound IV is carried out according to work-up procedures known per se, for example by means of customary filtration, crystallisation, distillation or chromatography procedures or any suitable combination thereof.

The yield obtainable for the reaction is generally very good. A yield of more than 85%, in many cases of more than 90%, of the theoretical yield is reached.

Preferred embodiments of the reaction are shown in the Examples P1.

The compound V is known. The compounds VI are known or can be prepared in analogy to corresponding known compounds.

The compound IV is a known compound and is disclosed, for example, in US-4,097,581 [hereinafter referred to as "(A)"] as intermediate useful in the preparation of agrochemicals. According to the prior art disclosure (A), the compound IV is manufactured by reaction of the compound V with 2,3-epoxypropan-1-ol. This prior art manufacturing process has, however, a great number of technical, ecological, economical and other disadvantages.

Thus, for example, the epoxy starting material used in the prior art process is only very difficult to handle. Being an extremely reactive compound, it is not very stable; therefore, the storage of 2,3-epoxypropan-1-ol has to be done with cooling. Such a cooling procedure, however, requires additional and rather complex technical equipment, in order to reach and maintain the necessary low temperatures. Such complex equipment, together with the energy required for the cooling process, make the storage of 2,3-epoxypropan-1-ol not only troublesome, but also very expensive and, consequently, economically uninteresting. Furthermore, the additional energy consumption associated with the cooling process is ecologically extremely undesired. Additionally, 2,3-epoxypropan-1-ol has a strong tendency to polymerise under the influence of catalysts, such a polymerisation being highly exothermal. This means, that the prior art manufacturing process, starting from 2,3-epoxypropan-1-ol, has to be carried out under special precautionary measures. For example, all of the equipment, such as reaction vessels, as well as all of the reagents and adjuvants, such as solvents, used in the course of those steps of the prior art manufacturing process where 2,3-epoxypropan-1-ol is present, must be employed in especially purified form, that is to say in a form being free of any 2,3-epoxypropan-1-ol polymerisation causing catalyst. However, the use of such especially purified equipment, reagents and adjuvants is not only very troublesome, but also extremely expensive and, consequently, makes the prior art process once again economically more uninteresting. Furthermore, the said polymerisation being exothermal, additional safety measures have to be taken, which allow to avoid the undesired effects caused by the heat freed during such a potential polymerisation reaction. Such safety measures are, for example, precautionary cooling measures and the reduction of the batch size. These additional anti-polymerisation safety measures necessary are again not only very troublesome, but also expensive and, consequently, make the prior art process once again economically less interesting. 2,3-Epoxypropan-1-ol, additionally, bears a number of considerable health hazards for the worker handling this substance. For example, it shows a strong irritating effect on the skin as well as on mucous membranes. Additionally, it can lead to allergies and can cause severe eye damage. 2,3-Epoxypropan-1-ol also acts on the central nervous system (CNS) by first inducing a CNS stimulation, followed by depression. The hereinbefore described, as well as further, health hazards make it necessary, to take appropriate additional safety measures in a manufacturing process making use of 2,3-epoxypropan-1-ol, which, in turn, again make such a process not only risky and troublesome, but also, due to the costs associated with the additional safety measures necessary, more expensive and, therefore, economically less interesting. Additionally, 2,3-epoxypropan-1-ol is a very expensive starting material. Its high price, therefore, makes the prior art manufacturing process for the compound IV even more expensive and, consequently, economically uninteresting. Furthermore, the prior art manufacturing process requires long reaction times. This means, that valuable manufacturing equipment is blocked for long periods of time, and, additionally, that the output in reaction product which can be reached per space of time is comparably very low. These disadvantageous properties make the prior art manufacturing process once again not only very troublesome, but also more expensive and, consequently, economically less interesting. Additionally, the yield in compound IV, obtained according to the prior art manufacturing process, is only low. This means, that disproportionate amounts of resources, such as reactants, adjuvants and energy, must be employed in the prior art process. This waste in resources makes the prior art manufacturing process not only very troublesome and ecologically disadvantageous, but also once again much more expensive and, consequently, economically uninteresting. The technical, ecological, economical and other disadvantages of the prior art process are not limited to the ones described hereinbefore, which latter ones are only intended to serve as a few examples for the great number of disadvantages inherent in the prior art process. All of the disadvantages of the prior art process mentioned hereinbefore cause severe problems already in the case, where the process is run in a laboratory scale. If the process is to be run, however, in a larger scale, these disadvantages cause difficulties which are enhanced to an even much greater extent, that is to say, it is almost impossible to run the prior art process in an industrial scale. To run a specific process in an industrial scale, however, is the final goal, if this process is intended for producing an intermediate for agrochemicals.

According to the process of the instant invention, the compound IV is manufactured by reaction of the compound V with a compound VI. This invention process has, highly surprisingly, a great number of technical, ecological, economical and other advantages, compared to the prior art process disclosed in (A). Thus, for example, the use of the disadvantageous epoxy starting material employed in the prior art process is avoided by replacing this epoxy material by the starting material VI. The starting material VI, being comparably stable, but being also still sufficiently reactive, is easy to handle. It can, for example, be stored without cooling. This means, that all of the disadvantages of the prior art process, associated with the necessity of storing the prior art epoxy starting material with cooling, are circumvented. The storage of the invention starting material VI is neither troublesome nor especially expensive, and, consequently, the invention process is, in this respect, economically much more interesting than the prior art process. Furthermore, there is no additional energy consumption, which would be associated with a cooling process, since there is no need to store the starting material VI with cooling, which also makes the invention process much more ecologically favourable, compared to the prior art process. Additionally, the starting material VI has no significant tendency to polymerise under the influence of catalysts. This means, that in the invention process there is no need for taking special precautionary measures, such as using equipment, reagents and adjuvants in an especially purified form being free of any polymerisation causing catalyst. Consequently, all of the disadvantages of the prior art process, associated with the necessity of taking such special precautionary measures, are circumvented, which, in turn, means, that the invention process can be run much more smoothly and much cheaper and, consequently, is, also in this respect, economically much more interesting than the prior art process. Furthermore, in the invention process there is no need for additional safety measures, such as precautionary cooling and reduction of the batch size, to be taken, which would allow to avoid the undesired effects caused by the heat freed during an exothermal polymerisation reaction of the invention starting material VI, since there is no such polymerisation to a significant extent, which, in turn, means, that the invention process can be run much more smoothly and much cheaper and, consequently, is, also in this respect, economically much more interesting than the prior art process. Additionally, no health hazards comparable to those associated with the use of 2,3-epoxypropan-1-ol are known in connection with the use of the invention starting material VI. This means, that it is not necessary, to take additional extraordinary safety measures for the worker handling the starting material VI according to the manufacturing process of the invention, which, in turn, again means, that the invention process can be run much more smoothly, with considerably less risk and much cheaper and, consequently, is, also in

this respect, economically much more interesting than the prior art process. Additionally, the invention starting material VI is much cheaper than the prior art epoxy starting material. The much lower price of the former, therefore, makes the invention manufacturing process for the compound IV even less expensive and, consequently, economically very interesting. Furthermore, the invention manufacturing process requires only short reaction times, compared to the prior art process. This means, that valuable manufacturing equipment is not blocked for long periods of time, but can be used very efficiently, and, additionally, that the output in reaction product IV which can be reached per space of time is also much higher, compared to the prior art process. These advantageous properties make the invention manufacturing process once again not only much less troublesome, but also much cheaper and, consequently, economically very interesting. Additionally, the yield in compound IV, obtainable according to the invention process, is very high, compared to the prior art process. This means, that only very favourable amounts of resources, such as reactants, adjuvants and energy, are necessary in the invention process. This very efficient use of the resources employed makes the invention manufacturing process not only very smooth and ecologically advantageous, but also once again much less expensive and, consequently, economically very interesting. The high yield in compound IV, obtainable according to the invention process, is extremely surprising, because it could be shown by means of analytical investigations, that in the course of the reaction according to the invention process 2,3-epoxypropan-1-ol, that is to say, the starting material according to the prior art process disclosed in (A), is formed, starting from the invention starting material VI, and that the 2,3-epoxypropan-1-ol thus formed subsequently reacts with the compound V. This means, that the invention starting material VI acts, in a certain sense, as a precursor for the prior art starting material 2,3-epoxypropan-1-ol, and, additionally, that by means of the invention process a much higher yield in product IV is obtainable, compared to the prior art process, although the invention process comprises an additional reaction step, that is to say, the formation of 2,3-epoxypropan-1-ol, starting from the invention starting material VI. The technical, ecological, economical and other advantages of the invention process are not limited to the ones described hereinbefore, which latter ones are only intended to serve as a few examples for the great number of advantages inherent in the invention process. With all of the advantages of the invention process mentioned hereinbefore, the severe problems encountered when carrying out the prior art process can be avoided already in the case, where the process is run in a laboratory scale. If the process is run, however, in a larger scale, these advantages are enhanced to have an even much greater effect, that is to say, only these advantages make it possible, to run the process in an industrial scale.

Therefore, by manufacturing the compound IV according to the process of the instant invention, all of the technical, ecological, economical and other disadvantages of the prior art process are advantageously overcome, which is indeed highly surprising.

Variant b):

Suitable acid catalysts for facilitating the reaction are, for example, strong inorganic acids, such as mineral acids, for example perchloric acid, sulfuric acid, nitric acid, nitrous acid, a phosphoric acid, such as phosphoric acid, or a hydrohalic acid, such as hydrochloric acid, strong organic carboxylic acids, such as unsubstituted or substituted, for example halogen-substituted, $C_1$-$C_4$alkanecarboxylic acids, for example acetic acid or trifluoroacetic acid, or unsaturated or saturated dicarboxylic acids, for example oxalic acid, malonic acid, succinic acid, maleic acid, fumaric acid or phthalic acid, or hydroxycarboxylic acids, for example ascorbic acid, lactic acid, malic acid, tartaric acid or citric acid, or benzoic acid, organic sulfonic acids, such as unsubstituted or substituted, for example halogen-substituted, $C_1$-$C_4$alkane- or arylsulfonic acids, for example methane sulfonic acid or p-toluenesulfonic acid, or camphor-10-sulfonic acid, acidic ion exchanger resins containing sulpho groups, Lewis acids, such as boron trifluoride-diethyl ether or boron trifluoride-dimethyl ether complexes, and acidic argillaceous earths, such as those from the group of the bentonites. Among these acid catalysts preferred are mineral acids, organic sulfonic acids, acidic ion exchanger resins containing sulpho groups, Lewis acids and acidic argillaceous earths. Especially preferred are methane sulfonic acid, p-toluenesulfonic acid and camphor-10-sulfonic acid. Very especially preferred is p-toluenesulfonic acid. In the reaction, the acid catalyst is preferably used in an amount of from 0.02 to 5 percent by mole, more preferably of from 0.2 to 1 percent by mole, relative to the amount of compound IV used.

During the reaction, for example from 0 to 90%, preferably from 0 to 50%, more preferably from 0 to 10%, even more preferably from 0 to 2%, most preferably 0%, of the water formed during the reaction are removed. If a part of the water formed during the reaction is removed (according to all embodiments of the invention process, a certain amount, for example at least 10%, most preferably 100%, of the water formed during the reaction must remain in the reaction mixture), this removal is preferably accomplished by customary procedures, for example by, for example continuous, preferably azeotropic, distillation.

The reactants can be reacted with each other as such, i. e. without adding a solvent or diluent, for example

in the molten state. However, in most cases it is advantageous to add an inert solvent or diluent or a mixture of these. The following may be mentioned as examples of such solvents or diluents: aromatic, aliphatic and alicyclic hydrocarbons and halohydrocarbons, such as benzene, toluene, xylene, mesitylene, tetralin, chlorobenzene, dichlorobenzene, bromobenzene, petroleum ether, hexane, cyclohexane, dichloromethane, trichloromethane, tetrachloromethane, dichloroethane, trichloroethene or tetrachloroethene; and ethers, such as diethyl ether, dipropyl ether, diisopropyl ether, dibutyl ether, tert-butyl methyl ether, ethylene glycol dimethyl ether, dimethoxydiethyl ether, tetrahydrofuran or dioxane. If the acid catalyst employed is an acid which is liquid and inert (except for acting as an acid catalyst) under the reaction conditions, then such an acid which is employed in excess, for example a strong organic carboxylic acid, such as an unsubstituted or substituted, for example halogen-substituted, $C_1$-$C_4$alkanecarboxylic acid, for example acetic acid or trifluoroacetic acid, can also act as solvent or diluent. Preferred are aromatic hydrocarbons and halohydrocarbons. Especially preferred are benzene, toluene, xylene and chlorobenzene. More especially preferred are toluene and xylene. Very especially preferred is toluene. The quantity of the inert solvent or diluent or of the inert solvent or diluent mixture, which is used in the reaction, is not critical; however, preferably a large excess thereof is used.

The molar ratio, in which the two reactants IV and VII are used in the reaction, being defined as the quotient "number of moles used of the compound IV: number of moles used of the compound VII", is, for example, less than 1, preferably from 0.3 to 0.9, more preferably from 0.45 to 0.9, most preferably from 0.6 to 0.9.

The reaction is advantageously carried out in a temperature range from approximately 0°C to the reflux temperature of the reaction mixture, preferably from approximately 0°C to approximately +120°C, more preferably from approximately +40°C to approximately +100°C, in many cases from approximately +50°C to the reflux temperature of the reaction mixture.

The reaction is preferably carried out under normal pressure. If a part of the water formed during the reaction is removed during the reaction by distillation, this is preferably done under slightly reduced pressure, for example in a pressure range from approximately 0.2 bar to normal pressure, preferably from approximately 0.2 bar to approximately 0.5 bar.

The reaction time is not critical; however, preferably a reaction time in the range from approximately 30 minutes to approximately 8 hours, more preferably from approximately 1 hour to approximately 6 hours, most preferably from approximately 2 hours to approximately 5 hours, is used.

The isolation of the compound III is carried out according to work-up procedures known per se, for example by means of customary filtration, crystallisation, distillation or chromatography procedures or any suitable combination thereof.

The yield obtainable for the reaction is generally very good. A yield of more than 85%, in many cases of more than 90%, often of more than 92%, of the theoretical yield is reached.

The compound III, being a mixture of the four configurational isomers, is obtained according to the process as a mixture, in which the racemate of the compounds IIIa and IIIb is present preferably in an amount of from 70 to 85% by weight, more preferably from 75 to 82% by weight, relative to the total weight of this isomer mixture, the remainder of the mixture being the racemate of the compounds IIIc and IIId. The proportion of the racemate of the compounds IIIa and IIIb in this isomer mixture is proportional to the content in the reaction mixture of water formed during the reaction: if much water is removed during the reaction, the proportion of the racemate of the compounds IIIa and IIIb in the resulting isomer mixture is smaller than in cases where less water is removed; the maximum proportion of the racemate of the compounds IIIa and IIIb in the resulting isomer mixture is obtained, if no water at all is removed during the reaction.

Preferred embodiments of the reaction are shown in the Examples P2.

The compounds IV are known; they are preferably prepared according to the invention process variant a) described above. The compound VII is known.

Certain mixtures of all, or of some, of the compounds IIIa, IIIb, IIIc and IIId are known. For example, in US-4,097,581 [(A)] there is disclosed, without any further specification with respect to the specific content in the different isomers, a mixture of diastereoisomers falling under the general formula III [hereinafter referred to as "compound (M)"] as agrochemically active ingredient. Further investigations of the instant inventors have shown, that the compound (M) is a mixture of all of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 50 to 60% by weight, relative to the total weight of this isomer mixture, the remainder of this isomer mixture being the racemate of the compounds IIIc and IIId. Furthermore, in EP-A-0 501 912 [hereinafter referred to as "(B)"] there is disclosed a specific mixture [hereinafter referred to as "compound (S)"] of the *2R,4R*-isomer IIIc and the *2R,4S*-isomer IIIa, the latter isomer, which has, as has - to a slightly lesser extent than the *2R,4S*-isomer IIIa - the *2S,4R*-isomer IIIb, the biologically most favourable properties of the four possible configurational isomers, being highly preponderant, as agrochemically active ingredient. No other mixtures of all, or of some, of the compounds IIIa,

IIIb, IIIc and IIId are known, that is to say, the instant compound III, being a mixture of the four configurational isomers, in which isomer mixture the biologically preferred racemate of the compounds IIIa and IIIb [hereinafter referred to as "racemate (P)"] is present in an amount of from 65 to 90% by weight, relative to the total weight of this isomer mixture, the remainder of this isomer mixture being the racemate of the compounds IIIc and IIId, is novel, as is consequently also the process for the manufacture of the compound III. According to the prior art disclosure (A), the compound (M), in the form of a mixture of diastereoisomers, which can be separated by physical methods to yield the respective diastereoisomers, is manufactured by reaction of the compound IV with the compound VII, this reaction being carried out in the presence of an acid catalyst, using an excess of the compound VII or, preferably, equivalent amounts of the compounds IV and VII, the water formed during the reaction being distilled off, advantageously by azeotropic distillation. According to the prior art disclosure (B), the compound (S) is manufactured by specific stereoselective syntheses. These prior art manufacturing processes according to (A) and (B) have, however, in each case, a great number of technical, ecological, economical and other disadvantages. According to the prior art manufacturing process disclosed in (A), for example, the preferred racemate (P) is obtained, unless additional separation steps are performed, only in an amount of from 50 to 60% by weight, relative to the total weight of the isomer mixture, whereas a proportion as high as possible of this preferred racemate (P) in the total isomer mixture would be highly desirable, because of the improvement in biological activity associated with an increase in the said proportion. In order to increase the proportion of the preferred racemate (P), it would be necessary to apply complex physical and/or chemical separation techniques, such as fractional crystallisation, chromatography, if desired also on chiral stationary phases, and derivatisation with defined optically active auxiliary substances followed by separation of the resulting derivatives and cleavage of the auxiliary substances. These separation techniques are not only extremely troublesome, but also very expensive and, consequently, economically uninteresting; furthermore, they are also not suitable for the preparation of the preferred racemate (P) in an industrial scale. Additionally, to obtain acceptable yields in compound (M), the water formed during the reaction has to be removed according to the prior art process (A), which, however, is very disadvantageous, as, for example, the removal of the water requires not only additional and rather complex technical equipment, but also, due to the necessary distillation procedure, the consumption of enormous additional amounts of energy. The necessary removal of the water, therefore, makes the prior art process according to (A) not only troublesome, but also very expensive and, consequently, economically uninteresting. Furthermore, the additional energy consumption associated with the distillation process is ecologically extremely undesired. The prior art manufacturing process disclosed in (B), that is to say, the specific stereoselective syntheses of the compound (S), requires not only extremely expensive enantiomerically pure educts and/or, also extremely expensive, noble metal catalysts, but also specific complex equipment, such as autoclaves, and, furthermore, long reaction times. All these features make the prior art manufacturing process disclosed in (B) not only extremely troublesome, but also very expensive and, consequently, economically uninteresting; furthermore, this prior art process is also not suitable for the preparation of the compound (S) in an industrial scale.

According to the process of the instant invention, the compound III is manufactured by reaction of the compound IV with the compound VII, this reaction being carried out in the presence of an acid catalyst, using equivalent amounts of the compounds IV and VII or, preferably, an excess of the compound VII, the water formed during the reaction not being removed completely. This invention process has, highly surprisingly, a great number of technical, ecological, economical and other advantages, compared to the prior art processes disclosed in (A) and (B), since virtually all of the disadvantages of these prior art processes are completely overcome with the novel invention process according to variant b). These technical, ecological, economical and other advantages of the invention process are not limited to the ones described hereinbefore, which latter ones are only intended to serve as a few examples for the great number of advantages inherent in the invention process. With all of the advantages of the invention process mentioned hereinbefore, the severe problems encountered when carrying out the prior art processes can be avoided already in the case, where the process is run in a laboratory scale. If the process is run, however, in a larger scale, these advantages are enhanced to have an even much greater effect, that is to say, only these advantages make it possible, to run the process in an industrial scale.

Variant c):

The process according to variant c) is carried out in such a way, that first of all the process according to variant a) is carried out, preferably in the manner described above, except for the isolation of the resulting compound IV, and that subsequently the thus obtained compound IV is further reacted, without isolation, according to the process of variant b), preferably in the manner described above.

In other words this means, that variants a) and b) are each followed as described above, except that the

work-up procedure of variant a) is modified.

Accordingly, in one preferred embodiment of variant c), the variant a) is followed up to the point, where the compound IV is present in the organic phase which is, according to variant a), subsequently induced to crystallize (cf. Examples P1). This crystallization is, however, according to variant c) not carried out, but the resulting organic phase containing the compound IV is instead used, without further purification or isolation steps being performed, optionally after dilution to an appropriate volume, in the process according to variant b).

In another preferred embodiment of variant c), the variant a) is followed up to the point, where the compound IV is present in the form of a moist filter cake, made up of crystals of the compound IV, which filter cake is, according to variant a), subsequently dried (cf. Examples P1). This drying step is, however, according to variant c) not carried out, but the resulting moist filter cake is instead used in the process according to variant b).

Preferably the same solvent or diluent is used according to variant c) in the two reaction steps corresponding to the processes of variants a) and b).

The total yield obtainable in the process according to variant c) is generally very good. A total yield of more than 75%, in many cases of more than 80%, often of more than 85%, of the theoretical total yield is reached.

The compound III, being a mixture of the four configurational isomers, is obtained according to variant c) as a mixture, in which the racemate of the compounds IIIa and IIIb is present preferably in an amount of from 70 to 85% by weight, more preferably from 70 to 76% by weight, relative to the total weight of this isomer mixture, the remainder of the mixture being the racemate of the compounds IIIc and IIId.

Preferred embodiments of the reaction are shown in the Examples P3.

The process of the instant invention according to variant c), being , on principle, an advantageous combination of the invention process variants a) and b), combining the processes according to variants a) and b) in the sense of a one pot reaction, has, compared with the respective prior art processes according to (A) and (B) discussed in detail hereinbefore, all of the great number of advantages described hereinbefore for the invention processes according to variants a) and b). Additionally, however, the invention process according to variant c) has still further technical, ecological, economical and other advantages, associated with the specific inventive feature, according to which the initially formed compound IV is not isolated, but is further reacted in situ with the compound VII. The non-isolation of the compound IV is advantageous, for example, in that the compound IV, after it has been crystallised and filtered off, has not to be dried, which not only saves energy and other resources, but also improves the safety of the process enormously, since the danger of an explosion of the dry powdery product IV is completely overcome. The savings in resources, especially in equipment and energy, are even greater, if the crystallised product IV is not filtered off at all, and are extremely great, if the product IV is not even crystallised, but is used in the form of the solution, which results from the reaction of the compound V with a compound VI. It is especially advantageous, that, compared with the separately carried out processes according to the invention process variants a) and b), the total reaction time is much shorter according to the invention process variant c), which consequently results in a much higher output in reaction product III per space of time and in a much more efficient use of valuable manufacturing equipment. Additionally, the total yield in compound III obtainable according to invention process variant c) is surprisingly very good and is, compared with the combined yields of the separately carried out processes according to the invention process variants a) and b), very much in the same percentage range. Even more surprisingly, also the proportion of the preferred racemate of the compounds IIIa and IIIb obtainable according to invention process variant c) is very good and is, compared with the respective proportion obtainable according to invention process variant b), very much in the same percentage range. The technical, ecological, economical and other advantages of the invention process variant c) are not limited to the ones described hereinbefore, which latter ones are only intended to serve as a few examples for the great number of advantages inherent in the invention process variant c).

Therefore, by manufacturing the compound III according to the process variant c) of the instant invention, a great number of technical, ecological, economical and other advantages, which are indeed highly surprising, can be efficiently used.

The compounds I can also be obtained in the form of their hydrates and/or can also include other solvents, for example solvents which may be used for crystallising compounds in solid form.

The invention relates to all those embodiments of the process in which, starting from a starting material or intermediate which can be obtained in any desired step of the process, all or some of the missing steps are carried out or a starting material is used in the form of a derivative or salt thereof and/or the racemates or antipodes thereof or, in particular, formed under the reaction conditions.

The invention relates also to a compound of the formula III, being a mixture of the four configurational isomers, that is to say of the *2R,4S*-isomer IIIa, the *2S,4R*-isomer IIIb, the *2R,4R*-isomer IIIc and the *2S,4S*-isomer

IIId, in which isomer mixture the racemate of the compounds IIIa and IIIb is present in an amount of from 65 to 90% by weight, preferably from 70 to 85% by weight, more preferably from 75 to 82% by weight or from 70 to 76% by weight, relative to the total weight of this isomer mixture, the remainder of this isomer mixture being the racemate of the compounds IIIc and IIId.

As has been discussed in detail hereinbefore with respect to the invention process variant b), certain mixtures of all, or of some, of the compounds IIIa, IIIb, IIIc and IIId are known. These mixtures have been proposed in the literature as active ingredients in arthropodacidally active pesticides. However, the properties of these known mixtures, for example in terms of their biological activity or in terms of the economy of the procedures by means of which they are manufactured, are not entirely satisfactory, resulting in a demand for other pesticidally active ingredients, this object being achieved according to the invention by providing the present novel compounds III.

The compounds III according to the invention, which can be obtained as described above under the process variants b) and c), are active ingredients in the field of pesticides which have a very favourable biocidal spectrum and are valuable when used preventively and/or curatively even at low rates of application, while being well tolerated by warm-blooded species, fish and plants. The active ingredients according to the invention are effective against all or individual development stages of normally sensitive, but also resistant, animal pests, such as insects or representatives of the order Acarina. The insecticidal or acaricidal action of the active ingredients according to the invention can become apparent either directly, i.e. by destroying the pests, either immediately or only after some time has elapsed, for example during moulting, or indirectly, for example by a reduced oviposition and/or hatching rate where the good activity corresponds to a mortality rate of at least 50 to 60%.

Examples of the abovementioned animal pests are:

from the order Lepidoptera, for example,

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni and Yponomeuta spp.;

from the order Coleoptera, for example,

Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. and Trogoderma spp.;

from the order Orthoptera, for example,

Blatta spp., Blattella spp., Gryllotalpa spp., Leucophaea maderae, Locusta spp., Periplaneta spp. and Schistocerca spp.;

from the order Isoptera, for example,

Reticulitermes spp.;

from the order Psocoptera, for example,

Liposcelis spp.;

from the order Anoplura, for example,

Haematopinus spp., Linognathus spp., Pediculus spp., Pemphigus spp. and Phylloxera spp.;

from the order Mallophaga, for example,

Damalinea spp. and Trichodectes spp.;

from the order Thysanoptera, for example,

Frankliniella spp., Hercinothrips spp., Taeniothrips spp., Thrips palmi, Thrips tabaci and Scirtothrips aurantii;

from the order Heteroptera, for example,

Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. and Triatoma spp.;

from the order Homoptera, for example,

Aleurothrixus floccosus, Aleyrodes brassicae, Aonidiella spp., Aphididae, Aphis spp., Aspidiotus spp., Bemisia

tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae and Unaspis citri;
from the order Hymenoptera, for example,
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. and Vespa spp.;
from the order Diptera, for example,
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. and Tipula spp.;
from the order Siphonaptera, for example,
Ceratophyllus spp. and Xenopsylla cheopis;
from the order Thysanura, for example,
Lepisma saccharina; and
from the order Acarina, for example,
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. and Tetranychus spp..

The active ingredients according to the invention allow pests of the abovementioned type to be controlled, i.e. contained or destroyed, which occur in particular on plants, especially on useful plants and ornamentals in agriculture, horticulture and forests, or on parts of such plants, such as fruits, flowers, foliage, stalks, tubers or roots, and, in some cases, even newly-forming parts of the plants are still protected against these pests.

Suitable as target crops are, in particular, cereals, such as wheat, barley, rye, oats, rice, maize or sorghum; beets, such as sugar beet or fodder beet; fruit, for example pome fruit, stone fruit and soft fruit, such as apples, pears, plums, peaches, almonds, cherries, or berries, for example strawberries, raspberries or blackberries; leguminous plants, such as beans, lentils, peas or soya beans; oil crops, such as oilseed rape, mustard, poppies, olives, sunflowers, coconut, castor, cocoa, or groundnuts; cucurbits, such as pumpkins, cucumbers or melons; fibre plants, such as cotton, flax, hemp orjute; citrus fruits, such as oranges, lemons, grapefruit or tangerines; vegetables, such as spinach, lettuce, asparagus, cabbages, carrots, onions, tomatoes, potatoes or bell peppers; Lauraceae, such as avocado, cinnamon or camphor; and also tobacco, nuts, coffee, egg plants, sugar cane, tea, pepper, vines, hops, Musaceae, latex plants and ornamentals.

The active ingredients according to the invention are particularly suitable for controlling scale insects, such as Aonidiella aurantii, Ceroplastes floridensis, Ceroplastes sinensis, Lepidosaphes beckii, Lepidosaphes ulmi, Parlatoria pergandei, Planococcus citri, Quadraspidiotus perniciousus, Saissetia olea and Unaspis citri, in citrus and deciduous fruit crops, for controlling Adoxophyes orana, Cydia pomonella, Leucoptera scitella, Lobesia botrana and Psylla piricola in deciduous fruit crops and for controlling Nilaparvata lugens in rice crops.

Other fields of application for the active ingredients according to the invention are the protection of stored products and stores and of material and, in the hygiene sector, in particular the protection of domestic animals and productive livestock against pests of the abovementioned type. The active ingredients according to the invention are particularly suitable for controlling ticks, such as Boophilus microplus.

The invention therefore also relates to pesticides, such as emulsifiable concentrates, suspension concentrates, directly sprayable or dilutable solutions, spreadable pastes, dilute emulsions, wettable powders, soluble powders, dispersible powders, dusts, granules or encapsulations in polymeric substances, all of which comprise at least one of the active ingredients according to the invention and are to be selected depending on the intended aims and the prevailing circumstances.

In these compositions, the active ingredient is used as a pure active ingredient, for example a solid active ingredient in a specific particle size or, preferably, together with at least one of the auxiliaries conventionally used in the art of formulation, such as extenders, for example solvents or solid carriers, or such as surface-active compounds (surfactants).

Examples of suitable solvents are: unhydrogenated or partially hydrogenated aromatic hydrocarbons, preferably the fractions $C_8$ to $C_{12}$ of alkylbenzenes, such as xylene mixtures, alkylated naphthalenes or tetrahydronaphthalene, aliphatic or cycloaliphatic hydrocarbons, such as paraffins or cyclohexane, alcohols, such as ethanol, propanol or butanol, glycols and the ethers and esters thereof, such as propylene glycol, dipropylene glycol ether, ethylene glycol or ethylene glycol monomethyl ether or ethylene glycol monoethyl ether, ketones, such as cyclohexanone, isophorone or diacetanol alcohol, strongly polar solvents, such as N-methylpyrrolid-2-one, dimethyl sulfoxide or N,N-dimethylformamide, water, epoxidised or unepoxidised vegetable oils, such as epoxidised or unepoxidised rapeseed oil, castor oil, coconut oil or soya oil, and silicone oils.

Solid carriers which are used, for example for dusts and dispersible powders, are, as a rule, ground natural minerals, such as calcite, talc, kaolin, montmorillonite or attapulgite. To improve the physical properties, it is also possible to add highly-disperse silicas or highly-disperse absorptive polymers. Possible particulate, adsorptive carriers for granules are either porous types, such as pumice, brick grit, sepiolite or bentonite, or non-sorptive carrier materials, such as calcite or sand. In addition, a large number of granulated materials of inorganic or organic nature can be used, in particular dolomite or comminuted plant residues.

Depending on the nature of the active ingredient to be formulated, suitable surface-active compounds are non-ionic, cationic and/or anionic surfactants or surfactant mixtures which have good emulsifying, dispersing and wetting properties. The surfactants given hereinbelow are only to be regarded as examples; the specialist literature describes a large number of further surfactants conventionally used in the art of formulation and suitable according to the invention.

Suitable non-ionic surfactants are mainly polyglycol ether derivatives of aliphatic or cycloaliphatic alcohols, saturated or unsaturated fatty acids and alkylphenols which can have 3 to 30 glycol ether groups and 8 to 20 carbon atoms in the (aliphatic) hydrocarbon radical and 6 to 18 carbon atoms in the alkyl radical of the alkylphenols. Other suitable substances are water-soluble polyethylene oxide adducts with polypropylene glycol, ethylenediaminopolypropylene glycol and alkylpolypropylene glycol having 1 to 10 carbon atoms in the alkyl chain and 20 to 250 ethylene glycol ether groups and 10 to 100 propylene glycol ether groups. Conventionally, the abovementioned compounds contain 1 to 5 ethylene glycol units per propylene glycol unit. Examples which may be mentioned are nonylphenol polyethoxyethanols, castor oil polyglycol ethers, polypropylene/polyethylene oxide adducts, tributylphenoxypolyethoxyethanol, polyethylene glycol and octylphenoxypolyethoxyethanol. Fatty acid esters of polyoxyethylene sorbitan, such as polyoxyethylene sorbitan trioleate, are furthermore suitable.

The cationic surfactants are mainly quaternary ammonium salts which have at least one alkyl radical having 8 to 22 C atoms as substituent and, as further substituents, lower, free or halogenated, alkyl, benzyl or lower hydroxyalkyl radicals. The salts are preferably in the form of halides, methylsulfates or ethylsulfates. Examples are stearyltrimethylammonium chloride and benzyl-di(2-chloroethyl)ethylammonium bromide.

Suitable anionic surfactants can be either water-soluble soaps or water-soluble synthetic surface-active compounds. Soaps which are suitable are the alkali metal salts, alkaline earth metal salts and unsubstituted or substituted ammonium salts of higher fatty acids ($C_{10}$-$C_{22}$), such as the sodium salts or potassium salts of oleic or stearic acid, or of natural mixtures of fatty acids which can be obtained, for example, from coconut oil or tall oil; mention must also be made of the fatty acid methyltaurinates. However, synthetic surfactants are used more frequently, in particular fatty sulfonates, fatty sulfates, sulfonated benzimidazole derivatives or alkylarylsulfonates. The fatty sulfonates and fatty sulfates are, as a rule, in the form of alkali metal salts, alkaline earth metal salts or substituted or unsubstituted ammonium salts and have, as a rule, an alkyl radical having 8 to 22 C atoms, alkyl also including the alkyl moiety of acyl radicals; examples which may be mentioned are the sodium salt or potassium salt of ligninsulfonic acid, of the dodecylsulfuric ester or of a fatty alcohol sulfate mixture prepared from natural fatty acids. This group also includes the salts of the sulfuric esters and sulfonic acids of fatty alcohol/ethylene oxide adducts. The sulfonated benzimidazole derivatives preferably have two sulfonyl groups and one fatty acid radical having approximately 8 to 22 C atoms. Examples of alkylarylsulfonates are the sodium salts, calcium salts or triethanolammonium salts of dodecylbenzenesulfonic acid, of dibutylnaphthalenesulfonic acid or of a naphthalenesulfonic acid/formaldehyde condensation product. Other substances which are possible are suitable phosphates, such as salts of the phosphoric ester of a p-nonylphenol/(4-14) ethylene oxide adduct, or phospholipids.

As a rule, the compositions comprise 0.1 to 99%, in particular 0.1 to 95%, of active ingredient and 1 to 99.9%, in particular 5 to 99.9%, of at least one solid or liquid auxiliary, where, as a rule, 0 to 25%, in particular 0.1 to 20%, of the compositions can be surfactants (% in each case meaning per cent by weight). While concentrated compositions are more preferred as commercially available goods, the end consumer uses, as a rule, dilute compositions whose concentrations of active ingredient are considerably lower. Preferred compositions are, in particular, composed as follows (% = per cent by weight):

Emulsifiable concentrates:

| | |
|---|---|
| Active ingredient: | 1 to 90%, preferably 5 to 20% |
| Surfactant: | 1 to 30%, preferably 10 to 20 % |
| Solvent: | 5 to 98%, preferably 70 to 85% |

Dusts:

| | |
|---|---|
| Active ingredient: | 0.1 to 10%, preferably 0.1 to 1% |
| Solid carrier: | 99.9 to 90%, preferably 99.9 to 99% |

Suspension concentrates:

| | |
|---|---|
| Active ingredient: | 5 to 75%, preferably 10 to 50% |
| Water: | 94 to 24%, preferably 88 to 30% |
| Surfactant: | 1 to 40%, preferably 2 to 30% |

Wettable powders:

| | |
|---|---|
| Active ingredient: | 0.5 to 90%, preferably 1 to 80% |
| Surfactant: | 0.5 to 20%, preferably 1 to 15% |
| Solid carrier: | 5 to 99%, preferably 15 to 98% |

Granules:

| | |
|---|---|
| Active ingredient: | 0.5 to 30%, preferably 3 to 15% |
| Solid carrier: | 99.5 to 70%, preferably 97 to 85% |

The activity of the compositions according to the invention can be broadened considerably and adapted to prevailing circumstances by addition of other insecticidal or acaricidal active ingredients. Possible active ingredients which are added are, for example, representatives from the following classes of active ingredients: organophosphorus compounds, nitrophenols and derivatives, formamidines, ureas, carbamates, pyrethroids, chlorinated hydrocarbons and Bacillus thuringiensis preparations. The compositions according to the invention can also comprise other solid or liquid auxiliaries, such as stabilisers, for example epoxidised or unepoxidised vegetable oils (for example epoxidised coconut oil, rapeseed oil or soya oil), antifoams, for example silicone oil, preservatives, viscosity regulators, binders and/or tackifiers, and also fertilisers or other active ingredients for achieving specific effects, for example bactericides, fungicides, nematocides, molluscicides or selective herbicides.

The compositions according to the invention are prepared in a known manner, for example, in the absence of auxiliaries, by grinding, screening and/or compressing a solid active ingredient, or active ingredient mixture, for example to give a certain particle size, and, in the presence of at least one auxiliary, for example by intimately mixing and/or grinding the active ingredient, or active ingredient mixture, with the auxiliary(-ies). The invention also relates to these processes for the preparation of the compositions according to the invention and to the use of the compounds I for the preparation of these compositions.

The invention furthermore relates to the methods of application of the compositions, i.e. to the methods of controlling pests of the abovementioned type, such as spraying, atomising, dusting, brushing-on, seed-dressing, scattering or pouring, which are to be selected depending on the intended aims and prevailing circumstances, and to the use of the compositions for controlling pests of the abovementioned type. Characteristic rates of concentration are between 0.1 and 1000 ppm, preferably between 0.1 and 500 ppm, of active ingredient. The application rates per hectare are, as a rule, 1 to 2000 g of active ingredient per hectare, in particular 10 to 1000 g/ha, preferably 20 to 600 g/ha.

A preferred method of application in the field of crop protection is application to the foliage of the plants (foliar application) where frequency and rate of application will depend on the danger of infestation with the particular pest. However, the active ingredient can also reach the plants via the root system (systemic action), by drenching the locus of the plants with a liquid composition or by incorporating the active ingredient in solid form into the locus of the plants, for example into the soil, for example in the form of granules (soil application). In paddy rice, such granules can be metered to the flooded paddy field.

The compositions according to the invention are also suitable for protecting plant propagation material, for example seed, such as fruits, tubers or kernels, or plant cuttings, against animal pests. The propagation

material can be treated with the composition before planting, for example seed can be dressed before sowing. Alternatively, the active ingredients according to the invention can be applied to the seed kernels (coating), either by soaking the kernels in a liquid composition or by coating them with a solid composition. Alternatively, the composition can be applied to the site of planting when the propagation material is planted, for example it can be applied to the seed furrow during sowing. The invention furthermore relates to these methods for treating plant propagation material and the plant propagation material thus treated.

The Examples which follow are not limiting, but only intended to illustrate the invention. Temperatures are given in degrees celsius.

Preparation Examples

Examples P1:

1,2-Dihydroxy-3-(4-phenoxyphenoxy)-propane

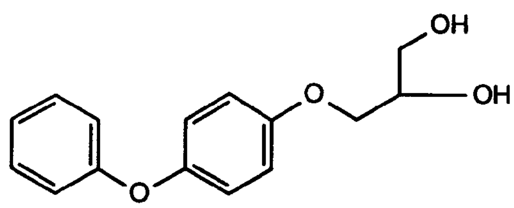

Example P1.1:

A suspension of 186.4 g (1 mol) of 4-phenoxyphenol, 207.6 g (1.5 mol) of potassium carbonate powder, 12 ml of water and 660 ml of toluene is heated to 90 to 95°. At this temperature, 124 g (1.12 mol) of 3-chloro-1,2-dihydroxy-propane are added to the suspension over a period of 2 hours. The reaction mixture is stirred for a further 2 hours at 90 to 95°, then cooled to 75 to 80° and, at this temperature, diluted with 400 ml of water. The aqueous phase is separated at 75 to 80°. The organic phase is then diluted again, using 145 ml of water, and the mixture is stirred for ten minutes at 75 to 80°. The aqueous phase is again separated at 75 to 80°. The organic phase is then heated to 110 to 115°, and the remainder of the water is removed by azeotropic distillation under normal pressure. The organic phase is then cooled to 55 to 60° and induced to crystallize. The crystallization starts at about 50 to 52°. The organic phase is then cooled to 20 to 25°, stirred for 5 hours at this temperature, then cooled to 0 to 5° and stirred for a further hour at this temperature. The crystalline product is filtered off with suction, washed twice, using for each washing 100 ml of toluene having a temperature of 0 to 5°, and dried under reduced pressure at a temperature of 50 to 60°. Thus, the title compound is obtained in the form of colourless crystals melting at 85 to 87°(yield: 92% of the theoretical yield).

Example P1.2:

The reaction is carried out in the same way as described in Example P1.1, with the exception, that 0.95 mol (instead of 1 mol) of 4-phenoxyphenol and 1.1 mol (instead of 1.12 mol) of 3-chloro-1,2-dihydroxy-propane are used, and with the further exception, that chlorobenzene (instead of toluene) is used. The title compound is obtained in the form of colourless crystals melting at 85 to 86°(yield: 89% of the theoretical yield).

Example P1.3:

The reaction is carried out in the same way as described in Example P1.1, with the exception, that 1.6 mol of sodium bicarbonate powder (instead of 1.5 mol of potassium carbonate powder) are used. The title compound is obtained in the form of colourless crystals melting at 85 to 87°(yield: 90% of the theoretical yield).

Example P1.4:

The reaction is carried out in the same way as described in Example P1.1, with the exception, that triethylamine (instead of potassium carbonate powder) is used. The title compound is obtained in the form of colourless crystals melting at 86 to 87°(yield: 89% of the theoretical yield).

Example P1.5:

A suspension of 186.4 g (1 mol) of 4-phenoxyphenol, 207.6 g (1.5 mol) of potassium carbonate powder, 12 ml of water and 700 ml of xylene (mixture of the isomers) is heated to 100 to 105°. At this temperature, 124 g (1.12 mol) of 3-chloro- 1,2-dihydroxy-propane are added to the suspension over a period of 2 hours. The reaction mixture is stirred for a further 2 hours at 100 to 105°, then cooled to 75 to 80° and, at this temperature, diluted with 400 ml of water. The aqueous phase is separated at 75 to 80°. The organic phase is then diluted again, using 145 ml of water, and the mixture is stirred for ten minutes at 75 to 80°. The aqueous phase is again separated at 75 to 80°. The organic phase is then heated to 135 to 150°, and the remainder of the water is removed by azeotropic distillation under normal pressure. The organic phase is then cooled to 60 to 65° and induced to crystallize. The crystallization starts at about 55 to 58°. The organic phase is then cooled to 20 to 25° and stirred for 15 hours at this temperature. The crystalline product is filtered off with suction, washed twice, using for each washing 80 ml of xylene (mixture of the isomers), and dried under reduced pressure at a temperature of 60 to 65°. Thus, the title compound is obtained in the form of colourless crystals melting at 84 to 86°(yield: 87% of the theoretical yield).

Example P1.6:

The reaction is carried out in the same way as described in Example P1.1, with the exception, that 1.55 mol of pyridine (instead of 1.5 mol of potassium carbonate powder) are used. The title compound is obtained in the form of colourless crystals melting at 85 to 86°(yield: 90% of the theoretical yield).

Example P1.7:

The reaction is carried out in the same way as described in Example P1.1, with the exception, that 3-bromo-1,2-dihydroxy-propane (instead of 3-chloro-1,2-dihydroxy-propane) is used. The title compound is obtained in the form of colourless crystals melting at 85 to 86°(yield: 88% of the theoretical yield).

Example P1.8:

A suspension of 186.4 g (1 mol) of 4-phenoxyphenol, 207.6 g (1.5 mol) of potassium carbonate powder, 12 ml of water and 750 ml of chlorobenzene is heated to 115 to 120°. At this temperature, 131.5 g (1.19 mol) of 3-chloro-1,2-dihydroxy-propane are added to the suspension over a period of 2.5 hours. The reaction mixture is stirred for a further 4 hours at 115 to 120°, then cooled to 75 to 80° and, at this temperature, diluted with 550 ml of water. The aqueous phase is separated at 75 to 80°. The organic phase is then diluted again, using 170 ml of water, and the mixture is stirred for ten minutes at 75 to 80°. The aqueous phase is again separated at 75 to 80°. The organic phase is then heated to 130 to 135°, and the remainder of the water is removed by azeotropic distillation under normal pressure. The organic phase is then cooled to 60 to 65° and induced to crystallize. The crystallization starts at about 54 to 56°. The organic phase is then cooled to 20 to 25° and stirred for 14 hours at this temperature. The crystalline product is filtered off with suction, washed with 90 ml of chlorobenzene and dried under reduced pressure at a temperature of 60 to 65°. Thus, the title compound is obtained in the form of colourless crystals melting at 85 to 86°(yield: 93% of the theoretical yield).

Example P1.9:

The reaction is carried out in the same way as described in Example P1.1, with the exception, that 1.6 mol of sodium methanolate powder (instead of 1.5 mol of potassium carbonate powder) are used. The title compound is obtained in the form of colourless crystals melting at 85 to 87°(yield: 91% of the theoretical yield).

Examples P2:

2-Ethyl-4-(4-phenoxyphenoxymethyl)-1,3-dioxolane (mixture of the four configurational isomers, comprising the *2R,4S*-isomer, the *2S,4R*-isomer, the *2R,4R*-isomer and the *2S,4S*-isomer)

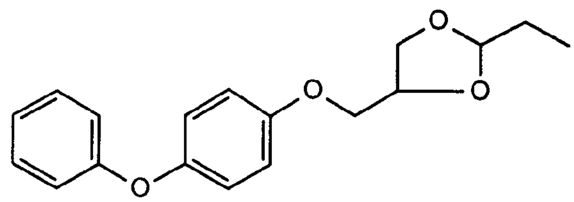

Example P2.1:

A solution of 91 g (0.35 mol) of 1,2-dihydroxy-3-(4-phenoxyphenoxy)-propane, 0.34 g (1.9 mmol) of p-toluenesulfonic acid and 300 ml of toluene is heated to 60 to 65°. At this temperature, 28 g (0.48 mol) of propanal are added to the solution over a period of 2 hours. The reaction mixture is stirred for a further hour at 60 to 65°, then, at this temperature, diluted with 100 ml of 1N aqueous sodium hydroxide solution and stirred for a further 15 minutes at 60 to 65°. The aqueous phase is separated at 60 to 65°. The organic phase is filtered through diatomaceous earth, and the filtrate is evaporated under reduced pressure. Thus, the title compound is obtained in the form of an oil (yield: 94% of the theoretical yield), comprising 37.8% of the *2R,4S*-isomer, 37.8% of the *2S,4R*-isomer, 12.2% of the *2R,4R*-isomer and 12.2% of the *2S,4S*-isomer. A sample of the dioxolane mixture is distilled under vacuum, and the product is found to boil at 180° (0.1 mbar).

Example P2.2:

The reaction is carried out in the same way as described in Example P2.1, with the exception, that 0.4 mol (instead of 0.48 mol) of propanal are used. The title compound is obtained in the form of an oil (yield: 88% of the theoretical yield), comprising 40.2% of the *2R,4S*-isomer, 40.2% of the *2S,4R*-isomer, 9.8% of the *2R,4R*-isomer and 9.8% of the *2S,4S*-isomer. A sample of the dioxolane mixture is distilled under vacuum, and the product is found to boil at 179° (0.1 mbar).

Example P2.3:

The reaction is carried out in the same way as described in Example P2.1, with the exception, that 2 mmol of camphor-10-sulfonic acid (instead of 1.9 mmol of p-toluenesulfonic acid) are used. The title compound is obtained in the form of an oil (yield: 93% of the theoretical yield), comprising 37.2% of the *2R,4S*-isomer, 37.2% of the *2S,4R*-isomer, 12.8% of the *2R,4R*-isomer and 12.8% of the *2S,4S*-isomer. A sample of the dioxolane mixture is distilled under vacuum, and the product is found to boil at 180° (0.1 mbar).

Example P2.4:

The reaction is carried out in the same way as described in Example P2.1, with the exception, that 0.3 mol (instead of 0.35 mol) of 1,2-dihydroxy-3-(4-phenoxy-phenoxy)-propane are used. The title compound is obtained in the form of an oil (yield: 91% of the theoretical yield), comprising 37.5% of the *2R,4S*-isomer, 37.5% of the *2S,4R*-isomer, 12.5% of the *2R,4R*-isomer and 12.5% of the *2S,4S*-isomer. A sample of the dioxolane mixture is distilled under vacuum, and the product is found to boil at 181° (0.1 mbar).

Example P2.5:

The reaction is carried out in the same way as described in Example P2.1, with the exception, that 1.8 mmol of methane sulfonic acid (instead of 1.9 mmol of p-toluenesulfonic acid) are used. The title compound is obtained in the form of an oil (yield: 92% of the theoretical yield), comprising 37.6% of the *2R,4S*-isomer, 37.6% of the *2S,4R*-isomer, 12.4% of the *2R,4R*-isomer and 12.4% of the *2S,4S*-isomer. A sample of the dioxolane mixture is distilled under vacuum, and the product is found to boil at 180° (0.1 mbar).

Example P2.6:

A solution of 91 g (0.35 mol) of 1,2-dihydroxy-3-(4-phenoxyphenoxy)-propane, 0.34 g (1.9 mmol) of p-toluenesulfonic acid and 350 ml of xylene (mixture of the isomers) is heated to 85 to 90°. At this temperature, 28 g (0.48 mol) of propanal are added to the solution over a period of 2 hours. The reaction mixture is stirred for a further 2 hours at 85 to 90°, then, at this temperature, diluted with 110 ml of 1N aqueous sodium hydroxide

solution and stirred for a further 15 minutes at 85 to 90°. The aqueous phase is separated at 85 to 90°. The organic phase is filtered through diatomaceous earth, and the filtrate is evaporated under reduced pressure. The title compound is obtained in the form of an oil (yield: 90% of the theoretical yield), comprising 36.9% of the *2R,4S*-isomer, 36.9% of the *2S,4R*-isomer, 13.1% of the *2R,4R*-isomer and 13.1% of the *2S,4S*-isomer. A sample of the dioxolane mixture is distilled under vacuum, and the product is found to boil at 179° (0.1 mbar).

Example P2.7:

A solution of 91 g (0.35 mol) of 1,2-dihydroxy-3-(4-phenoxyphenoxy)-propane, 0.34 g (1.9 mmol) of p-toluenesulfonic acid and 330 ml of chlorobenzene is heated to 75 to 80°. At this temperature, 28 g (0.48 mol) of propanal are added to the solution over a period of 2.5 hours. The reaction mixture is stirred for a further 2 hours at 75 to 80°, then, at this temperature, diluted with 100 ml of 1N aqueous sodium hydroxide solution and stirred for a further 20 minutes at 75 to 80°. The aqueous phase is separated at 75 to 80°. The organic phase is filtered through diatomaceous earth, and the filtrate is evaporated under reduced pressure. The title compound is obtained in the form of an oil (yield: 93% of the theoretical yield), comprising 36.4% of the *2R,4S*-isomer, 36.4% of the *2S,4R*-isomer, 13.6% of the *2R,4R*-isomer and 13.6% of the *2S,4S*-isomer. A sample of the dioxolane mixture is distilled under vacuum, and the product is found to boil at 180° (0.1 mbar).

Examples P3:

2-Ethyl-4-(4-phenoxyphenoxymethyl)-1,3-dioxolane (mixture of the four configurational isomers, comprising the *2R,4S*-isomer, the *2S,4R*-isomer, the *2R,4R*-isomer and the *2S,4S*-isomer)

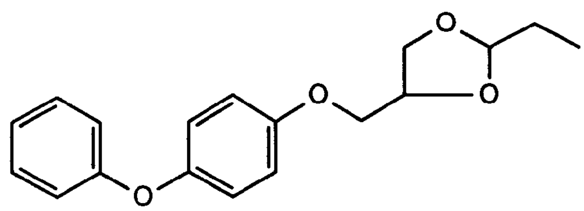

Example P3.1:

A suspension of 186.4 g (1 mol) of 4-phenoxyphenol, 207.6 g (1.5 mol) of potassium carbonate powder, 12 ml of water and 660 ml of toluene is heated to 90 to 95°. At this temperature, 124 g (1.12 mol) of 3-chloro-1,2-dihydroxy-propane are added to the suspension over a period of 2 hours. The reaction mixture is stirred for a further 2 hours at 90 to 95°, then cooled to 75 to 80° and, at this temperature, diluted with 400 ml of water. The aqueous phase is separated at 75 to 80°. The organic phase is then diluted again, using 145 ml of water, and the mixture is stirred for ten minutes at 75 to 80°. The aqueous phase is again separated at 75 to 80°. The organic phase is then heated to 110 to 115°, and the remainder of the water is removed by azeotropic distillation under normal pressure. The organic phase is then cooled to 55 to 60° and induced to crystallize. The crystallization starts at about 50 to 52°. The organic phase is then cooled to 20 to 25°, stirred for 5 hours at this temperature, then cooled to 0 to 5° and stirred for a further hour at this temperature. The crystalline product is filtered off with suction and washed twice, using for each washing 100 ml of toluene having a temperature of 0 to 5°. The moist filter cake is dissolved in 800 ml of toluene having a temperature of 60 to 65°. At this temperature, 0.89 g (5 mmol) of p-toluenesulfonic acid are added to the mixture, which is then stirred for 10 minutes at 60 to 65°. At the same temperature, 73.6 g (1.26 mol) of propanal are then added to the mixture over a period of 2 hours. The reaction mixture is stirred for a further hour at 60 to 65°, then, at this temperature, diluted with 270 ml of 1N aqueous sodium hydroxide solution and stirred for a further 20 minutes at 60 to 65°. The aqueous phase is separated at 60 to 65°. The organic phase is filtered through diatomaceous earth, and the filtrate is evaporated under reduced pressure. Thus, the title compound is obtained in the form of an oil (overall yield: 85% of the theoretical yield), comprising 37.5% of the *2R,4S*-isomer, 37.5% of the *2S,4R*-isomer, 12.5% of the *2R,4R*-isomer and 12.5% of the *2S,4S*-isomer. A sample of the dioxolane mixture is distilled under vacuum, and the product is found to boil at 180° (0.1 mbar).

Example P3.2:

A suspension of 186.4 g (1 mol) of 4-phenoxyphenol, 207.6 g (1.5 mol) of potassium carbonate powder, 12 ml of water and 700 ml of xylene (mixture of the isomers) is heated to 100 to 105°. At this temperature, 124 g (1.12 mol) of 3-chloro-1,2-dihydroxy-propane are added to the suspension over a period of 2 hours. The reaction mixture is stirred for a further 2 hours at 100 to 105°, then cooled to 75 to 80° and, at this temperature, diluted with 400 ml of water. The aqueous phase is separated at 75 to 80°. The organic phase is then diluted again, using 145 ml of water, and the mixture is stirred for ten minutes at 75 to 80°. The aqueous phase is again separated at 75 to 80°. The organic phase is then heated to 135 to 150°, and the remainder of the water is removed by azeotropic distillation under normal pressure. The organic phase is then cooled to 60 to 65° and induced to crystallize. The crystallization starts at about 55 to 58°. The organic phase is then cooled to 20 to 25° and stirred for 15 hours at this temperature. The crystalline product is filtered off with suction and washed twice, using for each washing 80 ml of xylene (mixture of the isomers). The moist filter cake is dissolved in 870 ml of xylene (mixture of the isomers) having a temperature of 85 to 90°. At this temperature, 0.85 g (4.7 mmol) of p-toluenesulfonic acid are added to the mixture, which is then stirred for 10 minutes at 85 to 90°. At the same temperature, 69.6 g (1.19 mol) of propanal are then added to the mixture over a period of 2 hours. The reaction mixture is stirred for a further 2 hours at 85 to 90°, then, at this temperature, diluted with 275 ml of 1N aqueous sodium hydroxide solution and stirred for a further 15 minutes at 85 to 90°. The aqueous phase is separated at 85 to 90°. The organic phase is filtered through diatomaceous earth, and the filtrate is evaporated under reduced pressure. Thus, the title compound is obtained in the form of an oil (overall yield: 77% of the theoretical yield), comprising 36.7% of the *2R,4S*-isomer, 36.7% of the *2S,4R*-isomer, 13.3% of the *2R,4R*-isomer and 13.3% of the *2S,4S*-isomer. A sample of the dioxolane mixture is distilled under vacuum, and the product is found to boil at 179° (0.1 mbar).

Example P3.3:

A suspension of 186.4 g (1 mol) of 4-phenoxyphenol, 207.6 g (1.5 mol) of potassium carbonate powder, 12 ml of water and 750 ml of chlorobenzene is heated to 115 to 120°. At this temperature, 131.5 g (1.19 mol) of 3-chloro-1,2-dihydroxy-propane are added to the suspension over a period of 2.5 hours. The reaction mixture is stirred for a further 4 hours at 115 to 120°, then cooled to 75 to 80° and, at this temperature, diluted with 550 ml of water. The aqueous phase is separated at 75 to 80°. The organic phase is then diluted again, using 170 ml of water, and the mixture is stirred for ten minutes at 75 to 80°. The aqueous phase is again separated at 75 to 80°. The organic phase is then heated to 130 to 135°, and the remainder of the water is removed by azeotropic distillation under normal pressure. The organic phase is then cooled to 60 to 65° and induced to crystallize. The crystallization starts at about 54 to 56°. The organic phase is then cooled to 20 to 25° and stirred for 14 hours at this temperature. The crystalline product is filtered off with suction and washed with 90 ml of chlorobenzene. The moist filter cake is dissolved in 880 ml of chlorobenzene having a temperature of 75 to 80°. At this temperature, 0.9 g (5.1 mmol) of p-toluenesulfonic acid are added to the mixture, which is then stirred for 10 minutes at 75 to 80°. At the same temperature, 74.4 g (1.28 mol) of propanal are then added to the mixture over a period of 2.5 hours. The reaction mixture is stirred for a further 2 hours at 75 to 80°, then, at this temperature, diluted with 100 ml of 1N aqueous sodium hydroxide solution and stirred for a further 20 minutes at 75 to 80°. The aqueous phase is separated at 75 to 80°. The organic phase is filtered through diatomaceous earth, and the filtrate is evaporated under reduced pressure. Thus, the title compound is obtained in the form of an oil (overall yield: 86% of the theoretical yield), comprising 36.2% of the *2R,4S*-isomer, 36.2% of the *2S,4R*-isomer, 13.8% of the *2R,4R*-isomer and 13.8% of the *2S,4S*-isomer. A sample of the dioxolane mixture is distilled under vacuum, and the product is found to boil at 180° (0.1 mbar).

Example P3.4:

A suspension of 186.4 g (1 mol) of 4-phenoxyphenol, 207.6 g (1.5 mol) of potassium carbonate powder, 12 ml of water and 660 ml of toluene is heated to 90 to 95°. At this temperature, 124 g (1.12 mol) of 3-chloro-1,2-dihydroxy-propane are added to the suspension over a period of 2 hours. The reaction mixture is stirred for a further 2 hours at 90 to 95°, then cooled to 75 to 80° and, at this temperature, diluted with 400 ml of water. The aqueous phase is separated at 75 to 80°. The organic phase is then diluted again, using 145 ml of water, and the mixture is stirred for ten minutes at 75 to 80°. The aqueous phase is again separated at 75 to 80°. The organic phase is then heated to 110 to 115°, and the remainder of the water is removed by azeotropic distillation under normal pressure. The organic phase is diluted with toluene to a volume of 1.1 1. At a temperature of 60 to 65°, 0.89 g (5 mmol) of p-toluenesulfonic acid are added to the organic phase, which is then stirred for 10

minutes. At the same temperature, 73.6 g (1.26 mol) of propanal are then added to the mixture over a period of 2 hours. The reaction mixture is stirred for a further hour at 60 to 65°, then, at this temperature, diluted with 270 ml of 1N aqueous sodium hydroxide solution and stirred for a further 20 minutes at 60 to 65°. The aqueous phase is separated at 60 to 65°. The organic phase is filtered through diatomaceous earth, and the filtrate is evaporated under reduced pressure. Thus, the title compound is obtained in the form of an oil (overall yield: 87% of the theoretical yield), comprising 37.9% of the *2R,4S*-isomer, 37.9% of the *2S,4R*-isomer, 12.1 % of the *2R,4R*-isomer and 12.1% of the *2S,4S*-isomer. A sample of the dioxolane mixture is distilled under vacuum, and the product is found to boil at 180° (0.1 mbar).

Example P3.5:

A suspension of 186.4 g (1 mol) of 4-phenoxyphenol, 207.6 g (1.5 mol) of potassium carbonate powder, 12 ml of water and 700 ml of xylene (mixture of the isomers) is heated to 100 to 105°. At this temperature, 124 g (1.12 mol) of 3-chloro-1,2-dihydroxy-propane are added to the suspension over a period of 2 hours. The reaction mixture is stirred for a further 2 hours at 100 to 105°, then cooled to 75 to 80° and, at this temperature, diluted with 400 ml of water. The aqueous phase is separated at 75 to 80°. The organic phase is then diluted again, using 145 ml of water, and the mixture is stirred for ten minutes at 75 to 80°. The aqueous phase is again separated at 75 to 80°. The organic phase is then heated to 135 to 150°, and the remainder of the water is removed by azeotropic distillation under normal pressure. The organic phase is diluted with xylene (mixture of the isomers) to a volume of 1.2 1. At a temperature of 85 to 90°, 0.85 g (4.7 mmol) of p-toluenesulfonic acid are added to the organic phase, which is then stirred for 10 minutes. At the same temperature, 69.6 g (1.19 mol) of propanal are then added to the mixture over a period of 2 hours. The reaction mixture is stirred for a further 2 hours at 85 to 90°, then, at this temperature, diluted with 275 ml of 1N aqueous sodium hydroxide solution and stirred for a further 15 minutes at 85 to 90°. The aqueous phase is separated at 85 to 90°. The organic phase is filtered through diatomaceous earth, and the filtrate is evaporated under reduced pressure. Thus, the title compound is obtained in the form of an oil (overall yield: 80% of the theoretical yield), comprising 36.4% of the *2R,4S*-isomer, 36.4% of the *2S,4R*-isomer, 13.6% of the *2R,4R*-isomer and 13.6% of the *2S,4S*-isomer. A sample of the dioxolane mixture is distilled under vacuum, and the product is found to boil at 179° (0.1 mbar).

Example P3.6:

A suspension of 186.4 g (1 mol) of 4-phenoxyphenol, 207.6 g (1.5 mol) of potassium carbonate powder, 12 ml of water and 750 ml of chlorobenzene is heated to 115 to 120°. At this temperature, 131.5 g (1.19 mol) of 3-chloro-1,2-dihydroxy-propane are added to the suspension over a period of 2.5 hours. The reaction mixture is stirred for a further 4 hours at 115 to 120°, then cooled to 75 to 80° and, at this temperature, diluted with 550 ml of water. The aqueous phase is separated at 75 to 80°. The organic phase is then diluted again, using 170 ml of water, and the mixture is stirred for ten minutes at 75 to 80°. The aqueous phase is again separated at 75 to 80°. The organic phase is then heated to 130 to 135°, and the remainder of the water is removed by azeotropic distillation under normal pressure. The organic phase is diluted with chlorobenzene to a volume of 1.2 1. At a temperature of 75 to 80°, 0.9 g (5.1 mmol) of p-toluenesulfonic acid are added to the organic phase, which is then stirred for 10 minutes. At the same temperature, 74.4 g (1.28 mol) of propanal are then added to the mixture over a period of 2.5 hours. The reaction mixture is stirred for a further 2 hours at 75 to 80°, then, at this temperature, diluted with 100 ml of 1N aqueous sodium hydroxide solution and stirred for a further 20 minutes at 75 to 80°. The aqueous phase is separated at 75 to 80°. The organic phase is filtered through diatomaceous earth, and the filtrate is evaporated under reduced pressure. Thus, the title compound is obtained in the form of an oil (overall yield: 87% of the theoretical yield), comprising 36.3% of the *2R,4S*-isomer, 36.3% of the *2S,4R*-isomer, 13.7% of the *2R,4R*-isomer and 13.7% of the *2S,4S*-isomer. A sample of the dioxolane mixture is distilled under vacuum, and the product is found to boil at 180° (0.1 mbar).

Formulation Examples (% = per cent by weight)

| Example F1: Emulsion concentrates | a) | b) | c) |
|---|---|---|---|
| Active ingredient | 25 % | 40 % | 50 % |
| Calcium dodecylbenzene sulfonate | 5 % | 8 % | 6 % |
| Castor oil polyethylene glycol ether (36 mol of EO) | 5 % | - | - |
| Tributylphenol polyethylene glycol ether (30 mol of EO) | - | 12 % | 4 % |
| Cyclohexanone | - | 15 % | 20 % |
| Xylene mixture | 65 % | 25 % | 20 % |

Emulsions of any desired concentration can be prepared from such concentrates by dilution with water.

| Example F2: Solutions | a) | b) | c) | d) |
|---|---|---|---|---|
| Active ingredient | 80 % | 10 % | 5 % | 95 % |
| Ethylene glycol monomethyl ether | 20 % | - | - | - |
| Polyethylene glycol MW 400 | - | 70 % | - | - |
| N-Methyl-2-pyrrolidone | - | 20 % | - | - |
| Epoxidised coconut oil | - | - | 1 % | 5 % |
| Petroleum spirit (boiling range 160-190°C) | - | - | 94 % | - |

The solutions are suitable for use in the form of microdrops.

| Example F3: Granules | a) | b) | c) | d) |
|---|---|---|---|---|
| Active ingredient | 5 % | 10 % | 8 % | 21 % |
| Kaolin | 94 % | - | 79 % | 54 % |
| Highly-disperse silica | 1 % | - | 13 % | 7 % |
| Attapulgite | - | 90 % | - | 18 % |

The active ingredient is dissolved in dichloromethane, the solution is sprayed onto the carrier, and the solvent is subsequently evaporated in vacuo.

| Example F4: Dusts | a) | b) |
|---|---|---|
| Active ingredient | 2 % | 5 % |
| Highly-disperse silica | 1 % | 5 % |
| Talc | 97 % | - |
| Kaolin | - | 90 % |

Ready-to-use dusts are obtained by intimately mixing the carriers with the active ingredient.

| Example F5: Wettable powders | a) | b) | c) |
|---|---|---|---|
| Active ingredient | 25 % | 50 % | 75 % |
| Sodium ligninsulfonate | 5 % | 5 % | - |
| Sodium lauryl sulfate | 3 % | - | 5 % |
| Sodium diisobutylnaphthalene-sulfonate | - | 6 % | 10 % |
| Octylphenol polyethylene glycol ether (7-8 mol of EO) | - | 2 % | - |
| Highly-disperse silica | 5 % | 10 % | 10 % |
| Kaolin | 62 % | 27 % | - |

The active ingredient is mixed with the additives, and the mixture is ground thoroughly in a suitable mill. This gives wettable powders which can be diluted with water to give suspensions of any desired concentration.

| Example F6: Emulsion concentrate | |
|---|---|
| Active ingredient | 10 % |
| Octylphenol polyethylene glycol ether | |
| (4-5 mol of EO) | 3 % |
| Calcium dodecylbenzene sulfonate | 3 % |
| Castor oil polyglycol ether | |
| (36 mol of EO) | 4 % |
| Cyclohexanone | 30 % |
| Xylene mixture | 50 % |

Emulsions of any desired concentration can be prepared from this concentrate by dilution with water.

| Example F7: Dusts | a) | b) |
|---|---|---|
| Active ingredient | 5 % | 8 % |
| Talc | 95 % | - |
| Kaolin | - | 92 % |

Ready-to-use dusts are obtained by mixing the active ingredient with the carrier and grinding the mixture on a suitable mill.

| Example F8: Extruder granules | |
|---|---|
| Active ingredient | 10 % |
| Sodium ligninsulfonate | 2 % |
| Carboxymethylcellulose | 1 % |
| Kaolin | 87 % |

The active ingredient is mixed with the additives, and the mixture is ground and moistened with water. This mixture is extruded, granulated and subsequently dried in a stream of air.

| Example F9: Coated granules | |
|---|---|
| Active ingredient | 3 % |
| Polyethylene glycol (MW 200) | 3 % |
| Kaolin | 94 % |

In a mixer, the finely ground active ingredient is applied uniformly to the polyethylene glycol, which has been moistened with kaolin. Dust-free coated granules are obtained in this manner.

| Example F10: Suspension concentrate | |
|---|---|
| Active ingredient | 40 % |
| Ethylene glycol | 10 % |
| Nonylphenol polyethylene glycol ether (15 mol of EO) | 6 % |
| Sodium ligninsulfonate | 10 % |
| Carboxymethylcellulose | 1 % |
| 37% aqueous formaldehyde solution | 0.2 % |
| Silicone oil in the form of a 75 % aqueous emulsion | 0.8 % |
| Water | 32 % |

The finely ground active ingredient is mixed intimately with the additives. This gives a suspension concentrate from which suspensions of any desired concentration can be prepared by dilution with water.

Biological Examples (% = per cent by weight, unless otherwise indicated)

The active ingredient is used, in each case, in three different isomer ratios A, B and C:
A = 37.8 % of *2R,4S*-isomer IIIa, 37.8% of *2S,4R*-isomer IIIb, 12.2% of *2R,4R*-isomer IIIc and 12.2% of *2S,4S*-isomer IIId;
B = 40.2% of *2R,4S*-isomer IIIa, 40.2% of *2S,4R*-isomer IIIb, 9.8% of *2R,4R*-isomer IIIc and 9.8% of *2S,4S*-isomer IIId; and
C = 36.7% of *2R,4S*-isomer IIIa, 36.7% of *2S,4R*-isomer IIIb, 13.3% of *2R,4R*-isomer IIIc and 13.3% of *2S,4S*-isomer IIId.

Example B1: Activity against Adoxophyes reticulana (ovicidal)

Adoxophyes reticulana eggs which have been deposited on filter paper are briefly immersed into a test solution of 400 ppm of active ingredient in acetone/water. After the test solution has dried on, the eggs are incubated in Petri dishes. After 6 days, the percentage hatching rate of the eggs is evaluated by comparison with untreated control batches (% reduction in hatching rate).
In this test, the compound of the formula III has an activity of over 80%.

Example B2: Activity against Aonidiella aurantii

Potato tubers are populated with Aonidiella aurantii crawlers. After about 2 weeks, the potatoes are immersed into an aqueous emulsion spray mixture or suspension spray mixture which comprises 400 ppm of active ingredient. After the tubers have dried, they are incubated in a plastic container. To evaluate the experiment after 10 to 12 weeks, the survival rate of the crawlers of the first subsequent generation of the treated population is compared with that of untreated control batches.
In this test, the compound of the formula III has an activity of over 80%.

Example B3: Activity against Aonidiella aurantii

Citrus trifoliata cuttings are populated with Aonidiella aurantii crawlers. After about 2 weeks, the cuttings are sprayed to drip point with an aqueous emulsion spray mixture comprising 50 ppm of active ingredient. To evaluate the experiment after 10 to 12 weeks, the survival rate of the crawlers of the first subsequent generation of the treated population is compared with that of untreated control batches.
In this test, the compound of the formula III has an activity of over 80%.

Example B4: Activity against Cydia pomonella (ovicidal)

Cydia pomonella eggs which have been deposited on filter paper are briefly immersed into a test solution of 400 ppm of active ingredient in acetone/water. After the test solution has dried on, the eggs are incubated in Petri dishes. After 6 days, the percentage hatching rate of the eggs is evaluated by comparison with untreated control batches (% reduction in hatching rate).
In this test, the compound of the formula III has an activity of over 80%.

Example B5: Activity against Nilaparvata lugens

Rice plants are sprayed with an aqueous emulsion spray mixture comprising 400 ppm of active ingredient. After the spray coating has dried on, the plants are populated with plant hopper larvae in the 2nd and 3rd stages. The test is evaluated after 21 days. The percentage reduction in population (% activity) is determined by comparing the number of surviving plant hoppers on the treated and untreated plants.
In this test, the compound of the formula III has an activity of over 80%.

Example B6: Activity against Nilaparvata lugens (systemic)

Pots containing rice plants are placed into an aqueous emulsion solution comprising 10 ppm of active ingredient. The plants are subsequently populated with larvae in the 2nd and 3rd stages. The test is evaluated after 6 days. The percentage reduction in population (% activity) is determined by comparing the number of plant hoppers on the treated and untreated plants.
In this test, the compound of the formula III has an activity of over 80%.

## Claims

1. A process for the manufacture of a compound of the formula

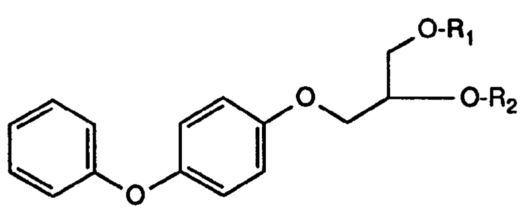

(I),

in which
either $R_1$ is hydrogen and $R_2$ is hydrogen
or O-$R_1$ and O-$R_2$ taken together are the group of the formula

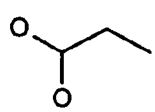

(II),

a compound of the formula I containing the group of the formula II, that is to say a 2-ethyl-4-(4-phenoxyphenoxymethyl)-1,3-dioxolane of the formula

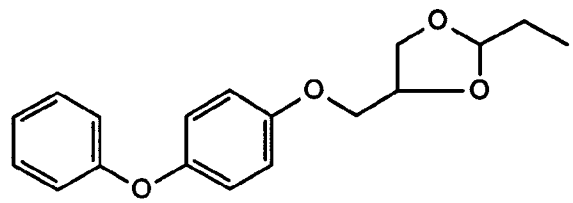

(III),

being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId,
which process is characterised in that

a) for the manufacture of a compound of the formula I, in which $R_1$ is hydrogen and $R_2$ is hydrogen, that is to say of a compound of the formula

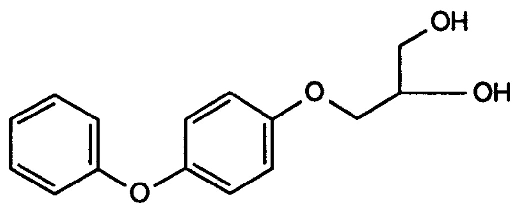

(IV),

the compound of the formula

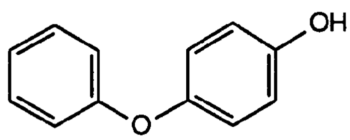

(V)

is reacted with a compound of the formula

$$Y\text{-}CH_2\text{-}CH(OH)\text{-}CH_2\text{-}OH \qquad (VI),$$

in which Y is a leaving group, or

b) for the manufacture of a compound of the formula III a compound of the formula IV is reacted with the compound of the formula

$$H_3C\text{-}CH_2\text{-}C(=O)\text{-}H \qquad (VII)$$

in the presence of an acid catalyst and without complete removal of the water formed during the reaction, the molar ratio, in which the two reactants are used, being defined as the quotient "number of moles used of the compound of the formula IV: number of moles used of the compound of the formula VII", being 1 or less than 1, or

c) for the manufacture of a compound of the formula III the compound of the formula V is reacted with a compound of the formula VI, in which Y is a leaving group, to give a compound of the formula IV, and this compound of the formula IV, without being isolated, is reacted with the compound of the formula VII in the presence of an acid catalyst and without complete removal of the water formed during the reaction, the molar ratio, in which the two reactants are used, being defined as the quotient "number of moles used of the compound of the formula IV: number of moles used of the compound of the formula VII", being 1 or less than 1.

**2.** A process according to claim 1 for the manufacture of a compound of the formula

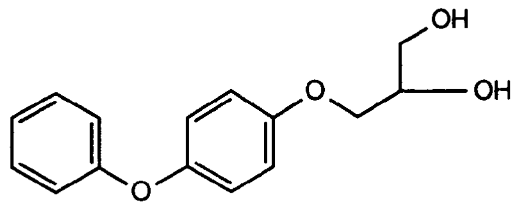

(IV),

characterised in that the compound of the formula

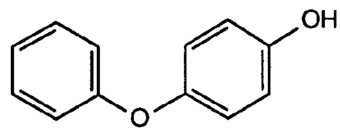

(V)

is reacted with a compound of the formula

$Y-CH_2-CH(OH)-CH_2-OH$ (VI),

in which Y is a leaving group.

3. A process according to claim 2, characterised in that a compound of the formula VI is used, in which Y is $C_1$-$C_8$alkoxy, halo-$C_1$-$C_8$alkoxy, $C_1$-$C_8$alkanoyloxy, $C_1$-$C_8$alkylthio, halo-$C_1$-$C_8$alkylthio, $C_1$-$C_8$alkanesulfonyloxy, halo-$C_1$-$C_8$alkanesulfonyloxy, benzenesulfonyloxy, toluenesulfonyloxy or halogen.

4. A process according to claim 3, characterised in that a compound of the formula VI is used, in which Y is chlorine.

5. A process according to claim 2, characterised in that the reaction is carried out in the presence of a base.

6. A process according to claim 5, characterised in that the base is selected from the group, consisting of an oxide, a hydroxide, a hydride, an amide, an alkanolate, an acetate, a carbonate, a bicarbonate, a dialkylamide or an alkylsilylamide of an alkali metal or of an alkaline earth metal, an alkylamine, an alkylenediamine, a free or N-alkylated, saturated or unsaturated, cycloalkylamine, a basic heterocycle and an ammonium hydroxide.

7. A process according to claim 6, characterised in that the base is potassium carbonate.

8. A process according to claim 7, characterised in that the base is potassium carbonate powder.

9. A process according to claim 5, characterised in that at least one equivalent of the base is used.

10. A process according to claim 9, characterised in that from 1.4 to 1.7 equivalents of the base are used.

11. A process according to claim 2, characterised in that the reaction is carried out in the presence of an inert solvent or diluent or a mixture of these.

12. A process according to claim 11, characterised in that the inert solvent or diluent is selected from the group consisting of benzene, toluene, xylene and chlorobenzene.

13. A process according to claim 12, characterised in that the inert solvent or diluent is toluene.

14. A process according to claim 13, characterised in that the inert solvent or diluent is toluene containing a catalytic amount of water.

15. A process according to claim 2, characterised in that the reactants V and VI are used in a molar ratio which is 1 or less than 1.

16. A process according to claim 15, characterised in that the reactants V and VI are used in a molar ratio which is from 0.8 to 0.95.

**17.** A process according to claim 2, characterised in that the reaction is carried out in a temperature range from 0°C to the reflux temperature of the reaction mixture.

**18.** A process according to claim 17, characterised in that the reaction is carried out in a temperature range from +70°C to the reflux temperature of the reaction mixture.

**19.** A process according to claim 2, characterised in that the reaction time is in the range from 30 minutes to 10 hours.

**20.** A process according to claim 1 for the manufacture of a compound of the formula

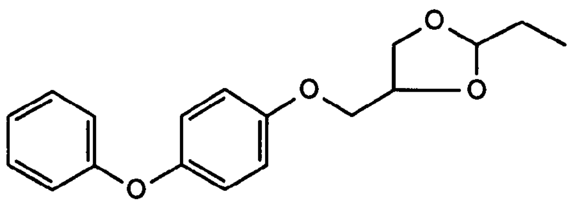

(III),

being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId, which process is characterised in that a compound of the formula

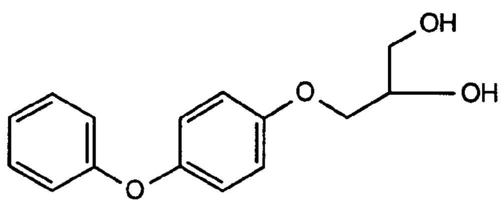

(IV)

is reacted with the compound of the formula

$H_3C-CH_2-C(=O)-H$ (VII)

in the presence of an acid catalyst and without complete removal of the water formed during the reaction, the molar ratio, in which the two reactants are used, being defined as the quotient "number of moles used of the compound of the formula IV: number of moles used of the compound of the formula VII", being 1 or less than 1.

**21.** A process according to claim 20, characterised in that the acid catalyst is selected from the group, consisting of the strong inorganic acids, the strong organic carboxylic acids, the organic sulfonic acids, the acidic ion exchanger resins containing sulpho groups, the Lewis acids and the acidic argillaceous earths.

**22.** A process according to claim 21, characterised in that the acid catalyst is selected from the group, consisting of methane sulfonic acid, p-toluenesulfonic acid and camphor-10-sulfonic acid.

**23.** A process according to claim 22, characterised in that the acid catalyst is p-toluenesulfonic acid.

**24.** A process according to claim 20, characterised in that the acid catalyst is used in an amount of from 0.02 to 5 percent by mole, relative to the amount of compound IV used.

**25.** A process according to claim 20, characterised in that from 0 to 90% of the water formed during the reaction are removed during the reaction.

**26.** A process according to claim 25, characterised in that from 0 to 2% of the water formed during the reaction are removed during the reaction.

**27.** A process according to claim 26, characterised in that 0% of the water formed during the reaction are removed during the reaction.

**28.** A process according to claim 20, characterised in that a part of the water formed during the reaction is removed during the reaction, this removal being accomplished by distillation.

**29.** A process according to claim 28, characterised in that the distillation is a continuous azeotropic distillation.

**30.** A process according to claim 20, characterised in that the reaction is carried out in the presence of an inert solvent or diluent or a mixture of these.

**31.** A process according to claim 30, characterised in that the inert solvent or diluent is toluene.

**32.** A process according to claim 20, characterised in that the reactants IV and VII are used in a molar ratio which is less than 1.

**33.** A process according to claim 32, characterised in that the reactants IV and VII are used in a molar ratio which is from 0.6 to 0.9.

**34.** A process according to claim 20, characterised in that the reaction is carried out in a temperature range from 0°C to the reflux temperature of the reaction mixture.

**35.** A process according to claim 34, characterised in that the reaction is carried out in a temperature range from +40°C to +100°C.

**36.** A process according to claim 28, characterised in that the distillation is done in a pressure range from approximately 0.2 bar to normal pressure.

**37.** A process according to claim 20, characterised in that the reaction time is in the range from 30 minutes to 8 hours.

**38.** A process according to claim 20, characterised in that the compound III is obtained as a mixture, in which the racemate of the compounds IIIa and IIIb is present in an amount of from 75 to 82% by weight, relative to the total weight of this isomer mixture.

**39.** A process according to claim 1 for the manufacture of a compound of the formula

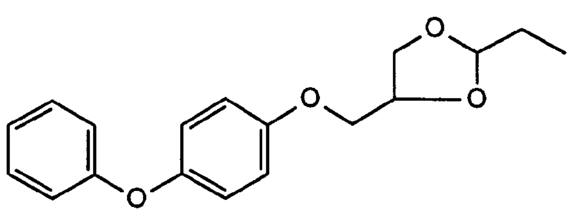

(III),

being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId, which process is characterised in that the compound of the formula

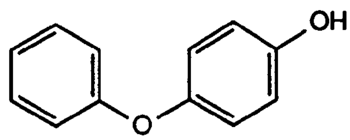

(V)

is reacted with a compound of the formula

$Y-CH_2-CH(OH)-CH_2-OH$ (VI),

in which Y is a leaving group, to give a compound of the formula

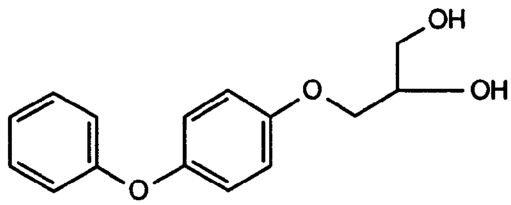

(IV),

and this compound of the formula IV, without being isolated, is reacted with the compound of the formula

$H_3C-CH_2-C(=O)-H$ (VII)

in the presence of an acid catalyst and without complete removal of the water formed during the reaction, the molar ratio, in which the two reactants are used, being defined as the quotient "number of moles used of the compound of the formula IV : number of moles used of the compound of the formula VII", being 1 or less than 1.

**40.** A process according to claim 39, characterised in that the reaction of the compound of the formula V with the compound of the formula VI is carried out as described in any one of claims 3 to 19.

**41.** A process according to claim 39, characterised in that the reaction of the compound of the formula IV with the compound of the formula VII is carried out as described in any one of claims 21 to 38.

**42.** A process according to claim 40, characterised in that the compound of the formula IV is obtained in the form of a solution in an organic solvent and is used in this form in the subsequent reaction with the compound of the formula VII.

**43.** A process according to claim 40, characterised in that the compound of the formula IV is obtained in the form of a moist filter cake and is used in this form in the subsequent reaction with the compound of the formula VII.

**44.** A process according to claim 39, characterised in that the reaction of the compound of the formula V with the compound of the formula VI and the reaction of the compound of the formula IV with the compound of the formula VII are carried out in the presence of the same solvent or diluent.

**45.** A process according to claim 39, characterised in that the compound III is obtained as a mixture, in which the racemate of the compounds IIIa and IIIb is present in an amount of from 70 to 76% by weight, relative to the total weight of this isomer mixture.

**46.** A compound of the formula

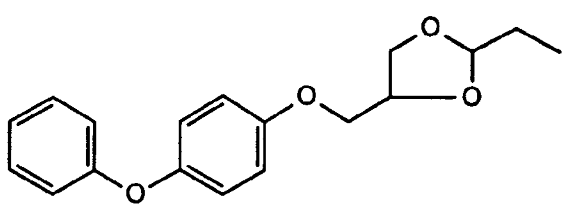

(III),

being a mixture of the four configurational isomers, that is to say of the *2R,4S*-isomer IIIa, the *2S,4R*-isomer IIIb, the *2R,4R*-isomer IIIc and the *2S,4S*-isomer IIId, in which isomer mixture the racemate of the compounds IIIa and IIIb is present in an amount of from 65 to 90% by weight, relative to the total weight of this isomer mixture, the remainder of this isomer mixture being the racemate of the compounds IIIc and IIId.

**47.** A compound according to claim 46 of the formula III, characterised in that the racemate of the compounds IIIa and IIIb is present in an amount of from 70 to 76% by weight, relative to the total weight of the isomer mixture.

**48.** A compound according to claim 46 of the formula III, characterised in that the racemate of the compounds IIIa and IIIb is present in an amount of from 75 to 82% by weight, relative to the total weight of the isomer

mixture.

**49.** A pesticidal composition which comprises at least one compound as claimed in claim 46 of the formula III as active ingredient and at least one auxiliary.

**50.** A process for the preparation of a composition as claimed in claim 49, characterised in that the active ingredient is mixed intimately and/or ground with the auxiliary(-ies).

**51.** Use of a compound as claimed in claim 46 of the formula III for the preparation of a composition as claimed in claim 49.

**52.** Use of a composition as claimed in claim 49 for controlling pests.

**53.** Use as claimed in claim 52 for protecting plant propagation material.

**54.** A method of controlling pests which comprises applying a composition as claimed in claim 49 to the pests or to their environment.

**55.** A method as claimed in claim 54 for the protection of plant propagation material, which comprises treating the propagation material or the locus where the propagation material is planted.

**56.** Plant propagation material treated by the method described in claim 55.

European Patent Office

# EUROPEAN SEARCH REPORT

Application Number
EP 94 81 0470

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | US-A-4 097 581 (FAROOQ) 27 June 1978<br>* column 10, line 30 - line 70; claims 1,15-18 * | 49-54 | C07D317/22<br>A01N43/28<br>C07C43/295<br>A01N31/14 |
| D,X | EP-A-0 501 912 (CIBA GEIGY AG) 2 September 1992<br>Example 1, page 11<br>* page 3, line 54 * | 46-54 | |
| X | EP-A-0 551 796 (CIBA GEIGY AG) 21 July 1993<br>* page 4, line 26 - line 27; claim 19 * | 49-54 | |
| P,X | GB-A-2 269 994 (CIBA GEIGY AG) 2 March 1994<br>* page 14, line 1 - line 6; claims 1-22 * | 49-54 | |

TECHNICAL FIELDS SEARCHED (Int.Cl.6)

C07D
A01N
C07C

The present search report has been drawn up for all claims

| Place of search | Date of completion of the search | Examiner |
|---|---|---|
| MUNICH | 19 December 1994 | Gettins, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)